(19) Europäisches Patentamt
European Patent Office
Office européen des brevets

(11) Numéro de publication: **0 253 738 B1**

(12) **FASCICULE DE BREVET EUROPEEN**

(45) Date de publication du fascicule du brevet: **31.01.90**

(51) Int. Cl.⁴: **C07D 305/14**, A61K 31/335

(21) Numéro de dépôt: **87401668.6**

(22) Date de dépôt: **16.07.87**

(54) **Dérivés du taxol, leur préparation et les compositions pharmaceutiques qui les contiennent.**

(30) Priorité: **17.07.86 FR 8610400**

(43) Date de publication de la demande: **20.01.88 Bulletin 88/3**

(45) Mention de la délivrance du brevet: **31.01.90 Bulletin 90/5**

(84) Etats contractants désignés: **AT BE CH DE ES FR GB GR IT LI LU NL SE**

(56) Documents cités:
**US-A- 4 206 221**

**COMPTES RENDUS DE L'ACADEMIE DES SCIENCES DE PARIS, t. 299, série II, no. 15, 1984, pages 1039-1042, Académie des Sciences, FR; V. SENILH et al.: "Hémisynthèse de nouveaux analogues du taxol. Etude de leur interaction avec la tubuline"**

(73) Titulaire: **RHONE-POULENC SANTE, 20, avenue Raymond Aron, F-92160 Antony(FR)**

(72) Inventeur: **Colin, Michel, 6 Grande Rue Auteuil le Roi, F-78770 Thoiry(FR)**
Inventeur: **Guenard, Daniel, 19 rue d'Arcueil, F-92120 Montrouge(FR)**
Inventeur: **Gueritte-Voegelein, Françoise, 201 rue Lecourbe, F-75015 Paris(FR)**
Inventeur: **Potier, Pierre, 14 avenue de Breteuil, F-75007 Paris(FR)**

(74) Mandataire: **Pilard, Jacques et al, RHONE-POULENC INTERSERVICES Service Brevets Pharma 25, Quai Paul Doumer, F-92408 Courbevoie Cédex(FR)**

**EP 0 253 738 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du taxol de formule générale :

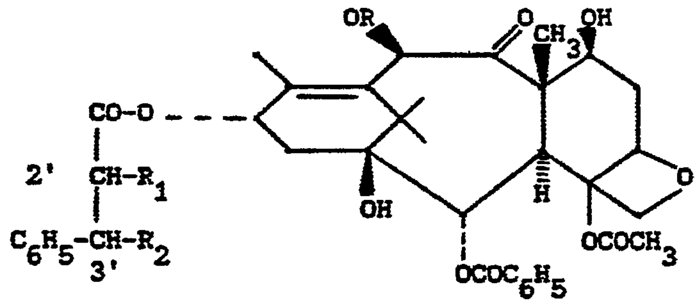

(I)

leur préparation et les compositions pharmaceutiques qui les contiennent.

Dans la formule générale (I), R représente un atome d'hydrogène ou un radical acétyle, l'un des symboles $R_1$ ou $R_2$ représente un radical hydroxy et l'autre représente un radical tertiobutoxycarbonylamino.

La présente invention concerne également les formes stéréoisomères des produits de formule générale (I) et leurs mélanges.

Le taxol, qui répond à la formule :

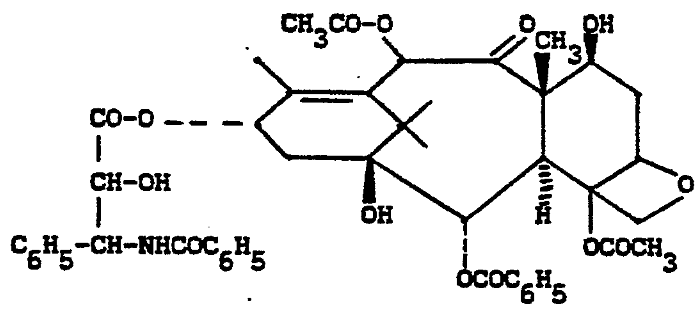

(II)

présente, in vitro des propriétés remarquables comme promoteur de la polymérisation de la tubuline et comme inhibiteur de la dépolymérisation des microtubules et, de ce fait, il constitue un agent antileucémique et antitumoral particulièrement intéressant.

Du fait de la difficulté d'extraction du taxol à partir des écorces de tronc de différentes espèces de Taxus, il a été proposé de préparer des dérivés analogues du taxol à partir de la désacétyl-10 baccatine III qui est extraite relativement facilement à partir des feuilles d'ifs. Cependant, les dérivés jusqu'à présent synthétisés ont montré une activité inférieure à celle du taxol [V. Senilh et coll., C.R. Acad. Sci., 299, série II, n° 15, p. 1039-1043 (1984)].

Il a maintenant été trouvé, et c'est ce qui fait l'objet de la présente invention, que les produits de formule générale (I) présentent une activité nettement supérieure à celle du taxol et, a fortiori, à celle des produits de formule générale (I) dans laquelle R représente un atome d'hydrogène, l'un des symboles $R_1$ ou $R_2$ représente un radical hydroxy et l'autre représente un radical éthoxycarbonylamino.

Selon la présente invention, les produits de formule générale (I) peuvent être obtenus par action du sel de sodium du N-chlorocarbamate de tertiobutyle sur un produit de formule générale :

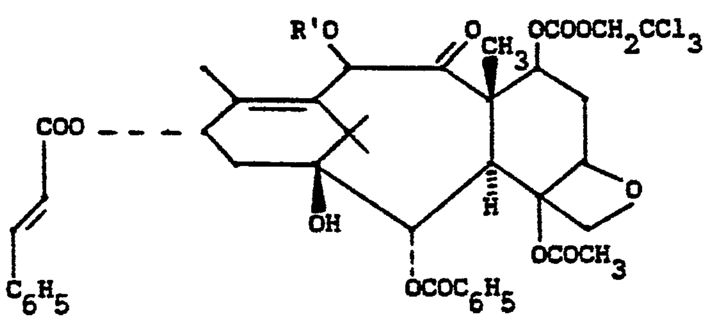

(III)

dans laquelle R′ représente un radical acétyle ou un radical trichloro-2,2,2 éthoxycarbonyle, en opérant dans un solvant organique tel que l'acétonitrile en présence de nitrate d'argent et d'une solution tertiobutanolique de tétraoxyde d'osmium à une température comprise entre 0 et 40°C, suivi du remplacement par un atome d'hydrogène du ou des groupements trichloro-2,2,2 éthoxycarbonyle du produit de formule générale :

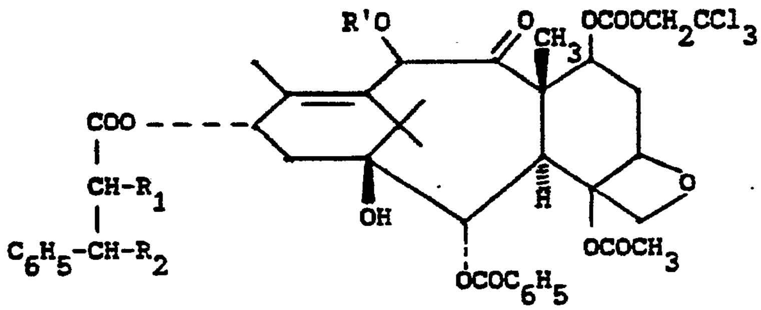

(IV)

dans laquelle R′, $R_1$ et $R_2$ sont définis comme précédemment au moyen de zinc en présence d'acide acétique à une température comprise entre 30 et 60°C.

L'action du sel de sodium du N-chlorocarbamate de tertiobutyle sur un produit de formule générale (III) conduit au mélange des isomères des produits de formule générale (IV) dont les constituants peuvent être séparés par des méthodes physico-chimiques telles que la chromatographie.

Le sel de sodium du N-chlorocarbamate de tertiobutyle peut être préparé à partir du carbamate de tertiobutyle selon la méthode décrite dans J. Amer. Chem. Soc., 100, 3596 (1978).

Le produit de formule générale (III) peut être obtenu par action du chlorure de cinnamoyle, éventuellement préparé in situ, sur le produit de formule générale :

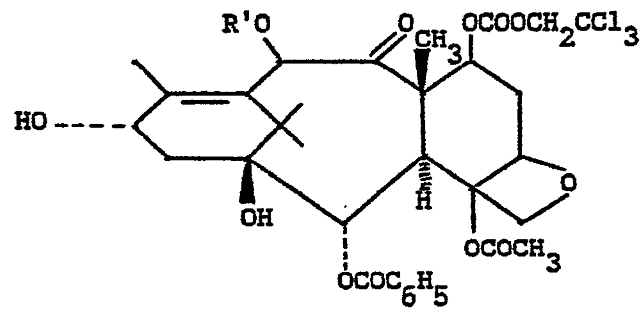

(V)

dans laquelle R′ est défini comme précédemment en opérant dans un solvant organique anhydre tel que le toluène en présence de cyanure d'argent à une température comprise entre 80 et 120°C.

Le produit de formule générale (III) peut aussi être obtenu par action de l'acide cinnamique sur le produit de formule générale (V) dans laquelle R′ est défini comme précédemment, en opérant dans un hydrocarbure aromatique tel que le benzène, le toluène ou les xylènes, en présence d'un agent de condensation tel qu'un carbodiimide comme le dicyclohexylcarbodiimide ou un carbonate réactif comme le dipyridyl-2 carbonate et d'un agent d'activation tel que la diméthylaminopyridine à une température comprise entre 60 et 90°C.

Par exemple, en opérant en présence de dicyclohexylcarbodiimide et de diméthylaminopyridine, il est particulièrement avantageux d'utiliser un excès molaire d'acide cinnamique par rapport au produit de formule générale (V), le dicyclohexylcarbodiimide étant utilisé en quantité stoechiométrique par rapport à l'acide cinnamique et la diméthylaminopyridine étant utilisée en quantité stoechiométrique par rapport au produit de formule (V). Généralement on utilise au moins 4 moles d'acide cinnamique par mole de produit de formule générale (V).

Le produit de formule générale (V) dans laquelle R′ est défini comme précédemment peut être obtenu par action du chloroformiate de trichloro-2,2,2 éthyle sur la baccatine III ou la désacétyl-10 baccatine III en opérant dans un solvant organique basique tel que la pyridine à une température comprise entre 0 et 50°C.

La baccatine III et la désacétyl-10 baccatine III sont des produits naturels qui peuvent être extraits à partir des feuilles ou de l'écorce d'ifs (Taxus baccata L).

Les produits de formule générale (I), et en particulier ceux pour lesquels R représente un atome d'hydrogène, $R_1$ représente un radical hydroxy et $R_2$ représente un radical tertiobutoxycarbonylamino, présentent des activités biologiques particulièrement intéressantes.

In vitro, la mesure de l'activité biologique est effectuée sur la tubuline extraite du cerveau de porc par la méthode de M.L. Shelanski et coll., Proc. Natl. Acad. Sci. U.S.A., 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude, les produits de formule générale (I) se sont montrés environ 2 fois plus actifs que le taxol.

In vivo, les produits formule générale (I) se sont montrés actifs chez la souris greffée par la leucémie L 1210 ou par la leucémie P 388 à des doses comprises entre 1 et 10 mg/kg par voie intrapéritonéale. A doses égales équitoxiques, les produits de formule générale (I) ont montré une efficacité antitumorale supérieure à celle du taxol (temps de survie augmenté, animaux survivants à long terme).

Les exemples suivants, donnés à titre non limitatif, montrent comment l'invention peut être mise en pratique.

EXEMPLE 1

On agite énergiquement pendant 5 minutes une solution de 0,5 g du sel de sodium du N-chlorocarbamate de tertiobutyle et de 1 g de nitrate d'argent dans 20 cm3 d'acétonitrile. On ajoute ensuite 0,2 cm3 d'une solution de tétraoxyde d'osmium dans l'alcool tertiobutylique (solution à 0,1 mole par litre), 2 g de produit de formule (III), dans laquelle R' représente un radical trichloro-2,2,2 éthoxycarbonyle, et 0,16 cm3 d'eau. Après 20 heures d'agitation à une température voisine de 20°C et à l'abri de la lumière, on ajoute 0,5 g de sel de sodium du N-chlorocarbamate de tertiobutyle, 0,1 cm3 de la solution de tétraoxyde d'osmium et 0,06 cm3 d'eau. Après 48 heures d'agitation énergique, le mélange réactionnel est filtré sur célite. Le filtre est rincé par de l'acétonitrile et le filtrat est concentré à sec. Le produit obtenu est purifié par chromatographie sur silice (silice Merck 7736) en éluant avec un mélange éther-hexane (50-50 en volumes) et en opérant sous légère pression. On isole de cette manière 900 mg de produit de formule (III) n'ayant pas réagi et les produits oxy-aminés qui sont purifiés et séparés par chromatographie sur couche épaisse (CCE) en éluant avec un mélange chlorure de méthylène-méthanol (98-2 en volumes).

On obtient ainsi :

- 295 mg de produit de formule générale (IV) dans laquelle R' représente un radical trichloro-2,2,2 éthoxycarbonyle, $R_1$ représente un radical hydroxy et $R_2$ représente un radical tertiobutoxycarbonylamino (2'R, 3'S) dont les caractéristiques sont les suivantes :
- pouvoir rotatoire : $[\alpha]_D^{23}$ = -38,4° (c = 1, chloroforme)
- spectre ultra-violet : λ max = 231 nm (15150)
λ max = 275 nm (1200)
λ max = 283 nm (1035)
- spectre infra-rouge : principales bandes d'absorption caractéristiques à 3850, 3440, 2960, 1770 et 1730 $cm^{-1}$
- spectre de résonance magnétique nucléaire du proton ($CDCl_3$ ; 400 MHz ; déplacements en ppm) : 1,21 (s, 3H) ; 1,27 (s, 3H) ; 1,36 (s, 9H) ; 1,86 (s, 3H) ; 1,96 (s, 3H) ; 2,39 (s, 3H) ; 2,62 (m, 1H) ; 3,90 (d, J = 7, 1H) ; 4,17 et 4,32 (2d, J = 9, 2H) ; 4,63 (d, J = 3, 1H) ; 4,59 et 4,90 (2d, J = 12, 2H) ; 4,77 (s, 2H) ; 4,96 (d, J = 9, 1H) ; 5,27 (dd, J = 9 et J = 3, 1H) ; 5,42 (d, J = 9, 1H) ; 5,55 (m, 1H) ; 5,69 (d, J = 7, 1H) ; 6.21 (t, J = 9, 1H) ; = 6,23 (s, 1H) ; 7.39 (5H); 7,51, 7,62 et 8,09 (5H).
- 250 mg de produit de formule générale (IV) dans laquelle R' représente un radical trichloro-2,2,2 éthoxycarbonyle, $R_1$ représente un radical hydroxy et $R_2$ représente un radical tertiobutoxycarbonylamino (2'S, 3'R) dont les caractéristiques sont les suivantes :
- pouvoir rotatoire : $[\alpha]_D^{23}$ = -43,5° (c = 1, chloroforme)
- spectre ultra-violet : λ max = 231 nm (15300)
λ max = 275 nm (1035)
λ max = 283 nm (905)
- spectre infra-rouge : bandes d'absorption caractéristiques à 3400, 3000, 1770 et 1730 $cm^{-1}$
- spectre de résonance magnétique nucléaire du proton ($CDCl_3$, 400 MHz, déplacements en ppm) : 1,18 (s, 3H) ; 1,23 (s, 3H) ; 1,40 (s, 9H) ; 1,86 (s, 3H) ; 2,08 (s, 3H) ; 2,24 (s, 3H) ; 2,64 (m, 1H) ; 3,98 (d, J = 7, 1H) 4,17 et 4.32 (d, J = 9, 2H) ; 4,48 (d, J = 3, 1H) ; 4,60 et 4,92 (2d, J = 12, 2H) ; 4,78 (s, 2H) ; 4,97 (d, J = 9, 1H) ; 5,22 (dd, J = 9 et J = 3, 1H) ; 5.32 (d, J = 9, 1H) ; 5,58 (m, 1H) ; 5,70 (d, J = 7, 1H) ; 6,07 (t, J = 9, 1H) ; 6,27 (s, 1H) ; 7,33-7,45 (5H) ; 7.48, 7,61 et 8,04 (5H).
- 250 mg de produit de formule générale (IV) dans laquelle R' représente un radical trichloro-2,2,2 éthoxycarbonyle, $R_1$ représente un radical tertiobutoxycarbonylamino et $R_2$ représente un radical hydroxy (2'R, 3'S) dont les caractéristiques sont les suivantes :
- pourvoir rotatoire $[\alpha]_D^{23}$ = -37,8° (c = 1, chloroforme)
- spectre ultra-violet : λ max = 231 nm (14500)
λ max = 274 nm (1730)
λ max = 282 nm (1520)
- spectre infra-rouge : bandes d'absorption caractéristiques à 3590, 3440, 3000, 1770 et 1730 $cm^{-1}$
- spectre de résonance magnétique nucléaire du proton ($CDCl_3$, 400 MHz, déplacements en ppm) : 1,20

(s, 3H) ; 1,27 (s, 3H) ; 1,37 (s, 9H) ; 1,87 (s, 3H) ; 2,02 (s, 3H) ; 2,42 (s, 3H) ; 2,64 (m, 1H) ; 3,96 (d, J = 7, 1H) ; 4,19 et 4,32 (2d, J = 9, 2H) ; 4,59 (d large, J = 12, 2H) ; 4,78 (s, 2H) ; 4,91 (d, J = 12, 1H) ; 5,00 (d, J = 9, 1H) ; 5,40 (s, 1H) ; 5,51 (d, J = 9, 1H) ; 5,58 (m, 1H) ; 5,69 (d, J = 7, 1H) ; 6,25 (s, 1H) ; 6,31 (t, J = 9, 1H) ; 7,36, 7,40 et 7,46 (5H) ; 7,48, 7,68 et 8,06 (5H).

- et 180 mg de produit de formule générale (IV) dans laquelle R′ représente un radical trichloro-2,2,2 éthoxycarbonyle, $R_1$ représente un radical tertiobutoxycarbonylamino et $R_2$ représente un radical hydroxy (2′S, 3′R) dont les caractéristiques sont les suivantes :

- pourvoir rotatoire : $[\alpha]_D^{23}$ = -32° (c = 1, chloroforme)

- spectre ultra-violet : λ max = 231 nm (14900)
λ max = 275 nm (1180)
λ max = 282 nm (1050)

- spectre infra-rouge : bandes d'absorption caractéristiques à 3600, 3440, 3000, 1770 et 1730 $cm^{-1}$

- spectre de résonance magnétique nucléaire du proton ($CDCl_3$, 400 MHz, déplacements en ppm) : 1,18 (s, 3H) ; 1,27 (s, 3H) ; 1,38 (s, 9H) ; 1,89 (s, 3H) ; 2,02 (s, 3H) ; 2,32 (s, 3H) ; 2,62 (m, 1H) ; 3,87 (d, J = 7, 1H) ; 4,15 et 4,32 (2d, J = 9, 2H) ; 4,60 (d large, J = 12, 2H) ; 4,77 (s, 2H) ; 4,91 (d, J = 12, 1H) ; 4,96 (d, J = 9, 1H) ; 5,16 (d, J = 3, 1H) ; 5,34 (d, J = 9, 1H) ; 5,57 (m, 1H) ; 5,67 (d, J = 7, 1H) ; 6,16 (t, J = 9, 1H) ; 6,23 (s, 1H) ; 7,39 (5H) ; 7,53, 7,66 et 8,07 (5H).

A une solution de 150 mg de produit de formule générale (IV) dans laquelle R′ représente un radical trichloro-2,2,2 éthoxycarbonyle, $R_1$ représente un radical hydroxy et $R_2$ représente un radical tertiobutoxycarbonylamino (2′R, 3′S) dans 5 cm3 d'acide acétique, on ajoute 150 mg de zinc en poudre. Le mélange réactionnel est agité pendant 2 heures à 50°C puis il est filtré et concentré à sec. On reprend le résidu par de l'eau puis on extrait à l'acétate d'éthyle. Les phases organiques réunies sont concentrées à sec et le résidu est purifié par chromatographie en couche épaisse en éluant avec un mélange chlorure de méthylène-méthanol (97-3 en volumes).

On obtient ainsi 94 mg de produit de formule générale (I) dans laquelle R représente un atome d'hydrogène, $R_1$ représente un radical hydroxy et $R_2$ représente un radical tertiobutoxycarbonylamino (2′R, 3′S) dont les caractéristiques sont les suivantes :

- pourvoir rotatoire : $[\alpha]_D^{23}$ = -36° (c = 0,74 ; éthanol)

- spectre ultra-violet : λ max = 230 nm (14800)
λ max = 275 nm (1730)
λ max = 283 nm (1670)

- spectre infra-rouge : principales bandes d'absorption caractéristiques à 3590, 3440, 1740-1700 $cm^{-1}$

- spectre de résonance magnétique nucléaire du proton ($CDCl_3$, 400 MHz, déplacements en ppm) : 1,12 (s, 3H) ; 1,24 (s, 3H) ; 1,35 (s, 9H) ; 1,77 (s, 3H) ; 1,87 (s, 3H) ; 2,28 (m, 2H) ; 2,37 (s, 3H) ; 2,58 (m, 1H) ; 3,91 (d, J = 7, 1H) ; 4,19 et 4,32 (2d, J = 9, 2H) ; 4,26 (m, 1H) ; 4,62 (d, J = 2, 1H) ; 4,94 (d, J = 9, 1H) ; 5,22 (s, 1H) ; 5,26 (dd, J = 9 et J = 2, 1H) ; 5,46 (d, J = 9, 1H) ; 5,68 (d, J = 7, 1H) ; 6,22 (t, J = 9, 1H) ; 7,38 (5H) ; 7,50, 7,60 et 8,12 (5H).

- spectre de masse (FAB) m/z : 808 ($MH^+$), 790, 752, 734, 708, 690, 527, 509, 449, 405, 387, 345, 327, 282, 226, 185.

Le produit de formule générale (III), dans laquelle R′ représente un radical trichloro-2,2,2 éthoxycarbonyle, peut être préparé selon l'une des méthodes suivantes :

1) A une solution de 9,84 g d'acide cinnamique (66,5 m.moles) dans 150 cm3 de toluène anhydre, on ajoute 11,92 cm3 de chlorure d'oxalyle. Le mélange réactionnel est agité pendant 1 heure à 60°C puis on élimine le chlorure d'oxalyle en excès par distillation. Le chlorure de cinnamoyle obtenu est repris par 300 cm3 de toluène anhydre puis on ajoute 12 g du produit de formule générale (V) dans laquelle R′ représente un radical trichloro-2,2,2 éthoxycarbonyle et 7,9 g de cyanure d'argent. Le mélange réactionnel est chauffé pendant 10 heures à 110°C sous agitation énergique. Après refroidissement, le mélange réactionnel est filtré et le précipité est rincé par de l'acétate d'éthyle. Les filtrats réunis sont versés dans de l'eau glacée. On extrait par de l'acétate d'éthyle. Les phases organiques réunies sont concentrées à sec puis reprises par 200 cm3 d'éther. Dans cette solution on fait passer un courant d'ammoniac jusqu'à précipitation du cinnamate d'ammonium formé. Après filtration la solution éthérée est concentrée et le résidu est chromatographie sur silice (silice Merck 7736) en éluant avec du chlorure de méthylène sous pression. On obtient ainsi avec un rendement de 55 %, 7,6 g du produit de formule générale (III) dans laquelle R′ représente un radical trichloro-2,2,2 éthoxycarbonyle dont les caractéristiques sont les suivantes :

- $[\alpha]_D$ = -56° (C = 0,567 ; chloroforme)

- spectre ultra-violet : λ max = 217 nm (26800)
λ max = 222 nm (26900)
λ max = 232 nm (16100)
λ max = 276 nm (23600)
λ max = 283 nm (24400)

- spectre infra-rouge : principales bandes d'absorption caractéristiques à 3420, 1760, 1725, 1710 et 1635 $cm^{-1}$

- spectre de résonance magnétique nucléaire du proton ($CDCl_3$ ; déplacements en ppm) : 5,73 (d, J = 7, $C_2H$) ; 3,99 (d, J = 7, $C_3H$) ; 5,02 (d, J = 9, $C_5H$); 1,88 et 2,68 (m, 2 x $C_6H$) ; 5,62 (m, $C_7H$) ; 6,30 (s, $C_{10}H$)

; 6,21 (t, J = 8, $C_{13}H$) ; 2,48 (m, $C_{14}H_2$) ; 1,29 (s, $C_{16}H_3$) ; 1,23 (s, $C_{17}H_3$) ; 2,16 (s, $C_{18}H_3$) ; 1,88 (s, $C_{19}H_3$) ; 4,20 et 4,34 (d, J = 9, 2 x $C_{20}H$) ; 2,31 (acétate) ; 7,45, 7,60 et 8,07 (benzoate) ; 6,53 (d, J = 16, $C_{2'}H$) ; 7,89 (d, J = 16, $C_{3'}H$) ; 7,45 (4H) ; 7,60 (1H) ; 4,62 à 4,93 (d, J = 12) ; 4,79 (s, 2H)
- spectre de masse (ionisation chimique) m/z 1023 ($MH^+$), 1005, 831, 813, 683, 665, 491, 431, 369, 309, 291, 149, 131, 123.

2) Dans un ballon tricol de 2 litres muni d'une agitation et d'un thermomètre, on introduit, sous atmosphère d'argon, 35,52 g d'acide cinnamique (240 m.moles), 1 litre de toluène anhydre, 49,44 g de dicyclohexylcarbodiimide (240 m.moles), 53,5 g de produit de formule générale (V) dans laquelle R' représente un radical trichloro-2,2,2 éthoxycarbonyle (60 m.moles) et 7,32 g de diméthylaminopyridine (60 m.moles). Le mélange est chauffé pendant 18 heures à 70°C sous atmosphère d'argon. Après refroidissement à 0°C pendant 4 heures, le précipité formé est séparé par filtration puis lavé par 100 cm3 de toluène froid.

Le filtrat est concentré à sec puis il est repris par 1 litre de chlorure de méthylène. La solution chlorométhylénique est lavée 3 fois par 150 cm3 d'une solution aqueuse d'acide chlorhydrique à 3 % (p/v). Après concentration de la phase organique, le résidu (92 g) est repris par 500 cm3 d'éther éthylique. La solution est laissée à une température voisine de 0°C pendant 48 heures. Le précipité formé est séparé par filtration et lavé par de l'éther éthylique à 0°C. Le filtrat est concentré à sec. On obtient ainsi 89 g d'un produit qui est chromatographié sur 2,7 kg de silice Merck 7734 en éluant avec un mélange toluène-méthanol (95-5 en volumes). On obtient ainsi, avec un rendement de 94,6 %, 58 g du produit de formule générale (III) dans laquelle R' représente un radical trichloro-2,2,2 éthoxycarbonyle.

Le produit de formule générale (V) dans laquelle R' représente un radical trichloro-2,2,2 éthoxycarbonyle peut être préparé de la manière suivante :

Une solution de 30 g de désacétyl-10 baccatine III (55 m.moles) dans 480 cm3 de pyridine anhydre est refroidie à 3°C sous atmosphère d'argon. On ajoute, en 3 minutes, 25,5 cm3 de chloroformiate de trichloro-2,2,2 éthyle (184 m.moles). Le mélange réactionnel est agité pendant 3 minutes à 20°C puis pendant 6 minutes à 28°C. La solution est ensuite refroidie au moyen d'un bain de glace puis elle est versée rapidement dans 1 litre d'eau glacée. La phase aqueuse est extraite en 3 fois par 1 litre au total de chlorure de méthylène. Après concentration, la pyridine est éliminée par épuisement au moyen de dichloro-1,2 éthane. Le produit brut obtenu (61,9 g) est purifié par chromatographie sur 1,2 kg de silice (silice Merck 7736) en éluant avec un mélange chlorure de méthylène-méthanol (99-1 en volumes).

On obtient ainsi, avec un rendement de 93 %, 45,6 g du produit de formule générale (V) dans laquelle R' représente un radical trichloro-2,2,2 éthoxycarbonyle dont les caractéristiques sont les suivantes :
- point de fusion : 233-234°C
- pouvoir rotatoire : $[\alpha]_D^{23}$ = -58° (C = 0,465 ; chloroforme)
- spectre ultra-violet : λ max = 232 nm (19000)
λ max = 276 nm (990)
λ max = 283 nm (810)
- spectre infra-rouge : bandes d'absorption caractéristiques à 3420, 1765, 1730 et 1720 $cm^{-1}$
- spectre de résonance magnétique nucléaire du proton ($CDCl_3$, déplacements en ppm) : 1,12 (s, 3H) ; 1,16 (s, 3H) ; 1,85 (s, 3H) ; 2,16 (s, 3H) ; 2,30 (s, 3H) ; 2,30 (m, 2H); 2,05 et 2,65 (2m, 2H) ; 4,00 (d, J = 7, 1H) ; 4, 18 et 4,35 (2d, J = 9, 2H ) ; 4, 63 et 4,92 (2d, J = 12, 2H) ; 4,76 et 4,80 (2d, J = 12, 2H) ; 4,92 (t, J = 9, 1H) ; 5,00 (d, J = 9, 1H) ; 5,61 (m, 1H) ; 5,66 (d, J = 7, 1H) ; 6,30 (s, 1H) ; 7,50, 7,64 et 8,13 (2t et 1d, J = 7, 5H)
- spectre de masse (ionisation chimique) m/z 893 ($MH^+$), 875, 701, 683, 579, 387, 327, 309, 123.

La désacétyl-10 baccatine III peut être obtenue de la manière suivante :

Des feuilles non séchées de Taxus baccata L (100 kg) sont broyées puis mises en percolation accélérée en tournaire avec de l'alcool à 95° (dont le taux réel en alcool passe à 80-85° du fait de l'eau contenue dans les feuilles). La première macération est effectuée avec 300 litres d'alcool et les macérations suivantes (4 fois 200 litres) sont effectuées avec de l'alcool récupéré par distillation et dont le degré alcoolique est maintenu à 85°. Chaque percolation dure 10 heures à une température voisine de 20°C. Le brassage est assuré par circulation de solvant au moyen d'une pompe.

Chaque phase éthanolique est concentrée sous pression réduite (50-60 mm de mercure ; 5,4 kPa). Les concentrats de chaque opération (70 litres environ), riches en eau, sont réunis et concentrés à nouveau jusqu'à un volume de 20 litres afin d'éliminer l'alcool résiduel.

L'extrait, qui n'est pas évaporé à sec, reste en milieu aqueux (20 litres) sous forme d'une suspension solide. On reprend par du chlorure de méthylène (9 extractions avec un total de 100 litres de chlorure de méthylène).

La solution chlorométhylénique ainsi obtenue (87 litres), qui contient 2 kg d'extrait sec, est concentrée jusqu'à un volume de 5 litres.

On chromatographie sur une colonne de 24 cm de diamètre contenant 10,3 kg de silice (zéosil : 8 kg ; célite : 2,3 kg).

On élue successivement, au débit de 8 à 9 litres/heure, avec :
- 150 litres de chlorure de méthylène (fraction 1)
- 150 litres d'un mélange chlorure de méthylène-méthanol (99,5-0,5 en volumes) (fraction 2)
- 170 litres d'un mélange chlorure de méthylène-méthanol (99-1 en volumes) (fraction 3)
- 130 litres d'un mélange chlorure de méthylène-méthanol (98-2 en volumes) (fraction 4)

Les deux premières fractions réunies fournissent 1,74 kg d'extrait sec. La troisième fraction fournit 390 g d'extrait sec. La quatrième fraction fournit 20 g d'extrait sec.

La troisième fraction (390 g), qui contient essentiellement la désacétyl-10 baccatine III, est à nouveau chromatographiée sur silice en éluant avec un mélange chlorure de méthylène-méthanol (99-1 en volumes) au débit de 4 litres/heure. On obtient ainsi 4 fractions dont la plus intéressante (154 g) conduit après concentration et digestion dans le chlorure de méthylène à 22 g de désacétyl-10 baccatine III pure.

Les eaux-mères (132 g), purifiées par chromatographie sur silice, fournissent 8 g de désacétyl-10 baccatine III.

Le rendement total est de 300 mg de désacétyl-10 baccatine III par kg de feuilles.

EXEMPLE 2

En opérant comme à l'exemple 1, mais à partir du produit de formule générale (IV) dans laquelle R' représente un radical trichloro-2,2,2 éthoxycarbonyle, $R_1$ représente un radical hydroxy et $R_2$ représente un radical tertiobutoxycarbonylamino (2'S, 3'R), on obtient le produit de formule générale (I) dans laquelle R représente un atome d'hydrogène, $R_1$ représente un radical hydroxy et $R_2$ représente un radical tertiobutoxycarbonylamino (2'S, 3'R) dont les caractéristiques sont les suivantes :

- pouvoir rotatoire : $[\alpha]_D^{23}$= -29° (c = 0,69 ; éthanol)
- spectre ultra-violet : λ max = 229 nm (14700)
λ max = 275 nm (2350)
λ max = 282 nm (2280)
- spectre infra-rouge : bandes d'absorption caractéristiques à 3580, 3440, 1740, 1700 $cm^{-1}$
- spectre de résonance magnétique nucléaire du proton ($CDCl_3$-$CD_3OD$, 400 MHz, déplacements en ppm) :1,14 (s, 3H) ; 1,20 (s, 3H) ; 1,40 (s, 9H) ; 1,75 (s, 3H) ; 1,97 (s, 3H) ; 2,27 (s, 3H) ; 2,53 (m, 1H) ; 3,90 (d, J = 7, 1H) ; 4,22 et 4,31 (2d, J = 9, 2H) ; 4,24 (m, 1H) ; 4,50 (d, J = 2, 1H) ; 5,01 (d, J = 9, 1H) ; 5,19 (d, J = 2, 1H) ; 5,32 (s, 1H) ; 5,67 (d, J = 7, 1H) ; 6,17 (t, J = 9, 1H) ; 7,26-7,45 (5H) ; 7,48, 7,62 et 8,07 (5H)
- spectre de masse (FAB) m/z : 808 ($MH^+$), 752, 734, 690, 527, 509, 449, 405, 387, 345, 327, 299, 266 et 185.

EXEMPLE 3

En opérant comme à l'exemple 1, mais à partir du produit de formule générale (IV) dans laquelle R' représente un radical trichloro-2,2,2 éthoxycarbonyle, $R_1$ représente un radical tertiobutoxycarbonylamino et $R_2$ représente un radical hydroxy (2'R, 3'S), on obtient le produit de formule générale (I) dans laquelle R représente un atome d'hydrogène, $R_1$ représente un radical tertiobutoxycarbonylamino et $R_2$ représente un radical hydroxy (2'R, 3'S) dont les caractéristiques sont les suivantes :

- pouvoir rotatoire : $[\alpha]_D^{23}$ = -29° (c = 0,47 ; éthanol)
- spectre ultra-voilet : λ max = 229 nm (16300)
λ max = 274 nm (2570)
λ max = 282 nm (2380)
-spectre infra-rouge : principales bandes d'absorption caractéristiques à 3590, 3440, 2990, 1740-1700 $cm^{-1}$
- spectre de résonance magnétique nucléaire du proton ($CDCl_3$, 400 MHz, déplacements en ppm) : 1,12 (s, 3H) ; 1,22 (s, 3H) ; 1,35 (s, 9H) ; 1,77 (s, 3H) ; 1,91 (s, 3H) ; 2,27 (m, 2H) ; 2,38 (s, 3H) ; 2,59 (m, 1H) ; 3,96 (d, J = 7, 1H) ; 4,19 et 4,31 (2d, J = 9, 2H) ; 4,25 (m, 1H) ; 4,58 (dd, J = 9 et J = 2, 1H) ; 4,97 (d, J = 9, 1H) ; 5,22 (s, 1H) ; 5,35 (d, J = 2, 1H) ; 5,48 (d, J = 9, 1H) ; 5,67 (d, J = 7, 1H) ; 6,26 (t, J = 9, 1H) ; 7,35, 7,40 et 7,46 (5H) ; 7,49, 7,62 et 8,07 (5H)
- spectre de masse (FAB) m/z : 808 ($MH^+$), 790, 752, 734, 708, 527, 509, 449, 405, 387, 345, 327, 282, 226 et 185.

EXEMPLE 4

En opérant comme dans l'exemple 1, mais à partir du produit de formule générale (IV) dans laquelle R' représente un radical trichloro-2,2,2 éthoxycarbonyle, $R_1$ représente un radical tertiobutoxycarbonylamino et $R_2$ représente un radical hydroxy (2'S, 3'R), on obtient le produit de formule générale (I) dans laquelle R représente un atome d'hydrogène, $R_1$ représente un radical tertiobutoxycarbonylamino et $R_2$ représente un radical hydroxy (2'S, 3'R) dont les caractéristiques sont les suivantes :

- pouvoir rotatoire : $[\alpha]_D^{23}$ = -33° (c = 0,81 ; éthanol)
- spectre ultra-voilet : λ max = 230 nm (14250)
λ max = 275 nm (1380)
λ max = 282 nm (1270)
- spectre infra-rouge : bandes d'absorption caractéristiques à 3580, 3440, 2900, 1740-1700 $cm^{-1}$
- spectre de résonance magnétique nucléaire du proton ($CDCl_3$, 400 MHz, déplacements en ppm) : 1,12

(s, 3H) ; 1,22 (s, 3H) ; 1,36 (s, 9H) ; 1,72 (s, 3H) ; 1,94 (s, 3H) ; 2,32 (s, 3H) ; 2,51 (m, 1H) ; 3,85 (d, J = 7, 1H) ; 4,20 et 4,29 (2d, J = 9, 2H) ; 4,22 (m, 1H) ; 4,58 (dd, J = 2 et J = 9, 1H) ; 4,97 (d, J = 9, 1H) ; 5,14 (d, J = 2, 1H) ; 5,22 (s, 1H) ; 5,65 (d, J = 7, 1H) ; 5,81 (d, J = 9, 1H) ; 6,17 (t, J = 9, 1H) ; 7,37 (5H) ; 7,50, 7,63 et 8,07 (5H)

- spectre de masse (FAB) m/z : 808 ($MH^+$), 752, 740, 708, 690, 549, 527, 509, 449, 405, 387, 345, 327, 299, 226 et 185.

La présente invention comprend également les compositions pharmaceutiques contenant les produits de formule générale (I) en association avec tout autre produit pharmaceutiquement acceptable qu'il soit inerte ou physiologiquement actif.

Ces compositions peuvent être présentées sous toute forme appropriée à la voie d'administration prévue. La voie parentérale est la voie d'administration préférentielle et notamment la voie intraveineuse.

Les compositions selon l'invention pour administration parentérale peuvent être des solutions stériles aqueuses ou non aqueuses, des suspensions ou des émulsions. Comme solvant ou véhicule, on peut employer le propylèneglycol, les huiles végétales, en particulier l'huile d'olive, et les esters organiques injectables, par exemple l'oléate d'éthyle. Ces compositions peuvent également comprendre des adjuvants en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également sous forme de compositions solides stériles qui peuvent être dissoutes ou dispersées dans de l'eau stérile ou tout autre milieu stérile injectable.

Les produits de formule générale (I) sont plus particulièrement utilisés dans le traitement des leucémies aigües et des tumeurs solides à des doses journalières généralement comprises entre 1 et 2 mg/kg par voie intraveineuse pour un adulte.

L'exemple suivant, donné à titre non limitatif, illustre une composition selon l'invention.

EXEMPLE

On dissout 40 mg du produit obtenu à l'exemple 1 dans 1 $cm^3$ d'Emulphor EL 620® et 1 $cm^3$ d'éthanol puis la solution est diluée par addition de 18 $cm^3$ de sérum physiologique.

La composition est administrée par introduction dans une perfusion d'un soluté physiologique pendant 1 heure.

**Revendications pour les états contractants: BE, CH, DE, FR, GB, IT, LI, LU, NL, SE**

1. Nouveau dérivé du taxol de formule générale :

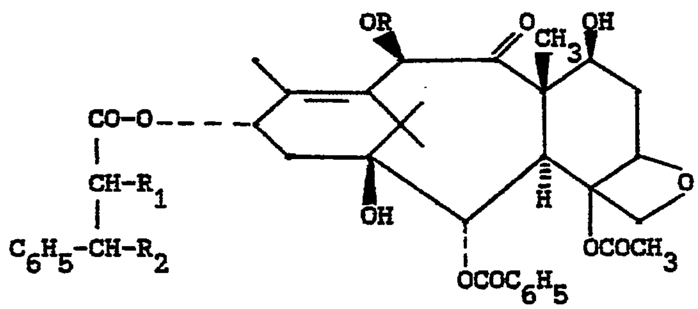

dans laquelle R représente un atome d'hydrogène ou un radical acétyle, l'un des symboles $R_1$ ou $R_2$ représente un radical hydroxy et l'autre représente un radical tertiobutoxycarbonylamino, ainsi que ses formes stéréoisomères et leurs mélanges.

2. Procédé de préparation d'un nouveau dérivé du taxol selon la revendication 1 caractérisé en ce que l'on fait agir le sel de sodium du N-chlorocarbamate de tertiobutyle sur un produit de formule générale:

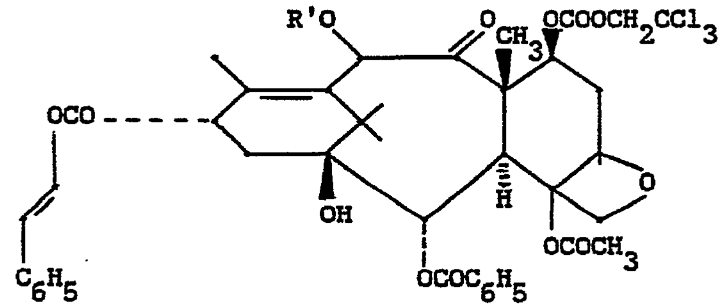

dans laquelle R′ représente un radical acétyle ou trichloro-2,2,2 éthoxycarbonyle, dans un solvant organique tel que l'acétonitrile en présence de nitrate d'argent et d'une solution tertiobutanolique de tétraoxyde d'osmium à une température comprise entre 0 et 40°C, puis remplace par un atome d'hydrogène le ou les groupements trichloro-2,2,2 éthoxycarbonyle du produit de formule générale:

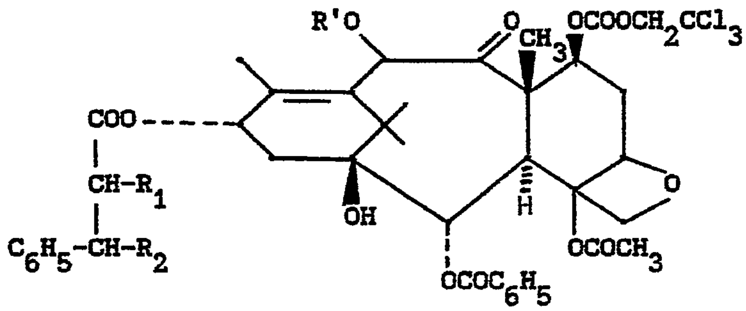

dans laquelle R′ est défini comme ci-dessus et $R_1$ et $R_2$ sont définis comme dans la revendication 1, au moyen de zinc en présence d'acide acétique à une température comprise entre 30 et 60°C, et isole le produit obtenu.

3. Procédé de préparation d'un produit de formule générale:

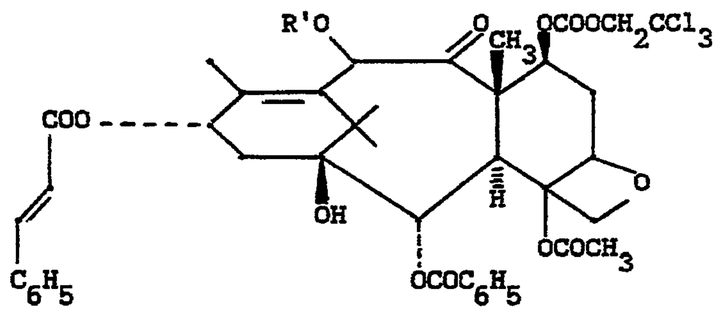

dans laquelle R′ représente un radical acétyle ou trichloro-2,2,2 éthoxycarbonyle caractérisé en ce que l'on fait réagir l'acide cinnamique sur un produit de formule générale:

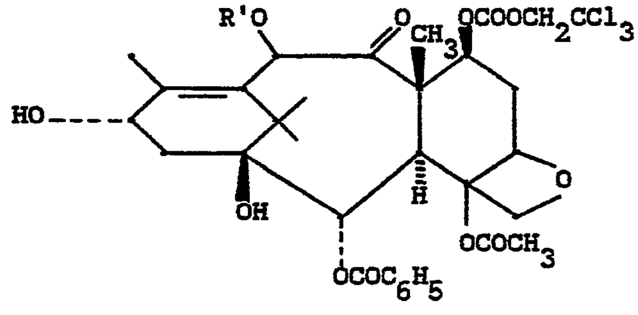

dans laquelle R′, est défini comme précédemment en opérant dans un hydrocarbure aromatique tel que le benzène, le toluène ou les xylènes en présence d'un agent de condensation tel qu'un carbodiimide comme

le dicyclohexylcarbodiimide ou un carbonate réactif comme le dipyridyl-2 carbonate et d'un agent d'activation tel que la diméthylaminopyridine à une température comprise entre 60 et 90°C.

4. Composition pharmaceutique caractérisée en ce qu'elle contient une quantité suffisante d'un produit selon la revendication 1 en association avec un ou plusieurs diluants ou adjuvants pharmaceutiquement acceptables, inertes ou pharmacologiquement actifs.

**Revendications pour les états contractants: AT, ES, GR**

1. Procédé de préparation d'un nouveau dérivé du taxol de formule générale:

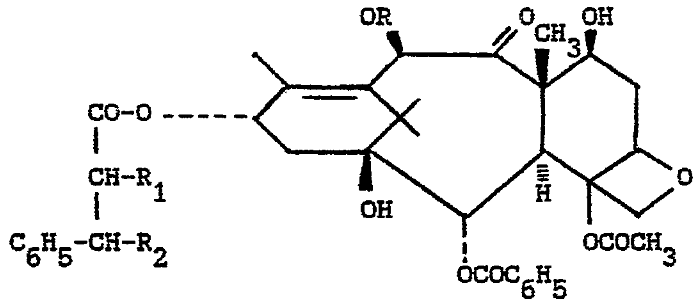

dans laquelle R représente un atome d'hydrogène ou un radical acétyle, l'un des symboles $R_1$ ou $R_2$ représente un radical hydroxy et l'autre représente un radical tertiobutoxycarbonylamino, ainsi que ses formes stéréoisomères et leurs mélanges, caractérisé en ce que l'on fait agir le sel de sodium du N-chlorocarbamate de tertiobutyle sur un produit de formule générale:

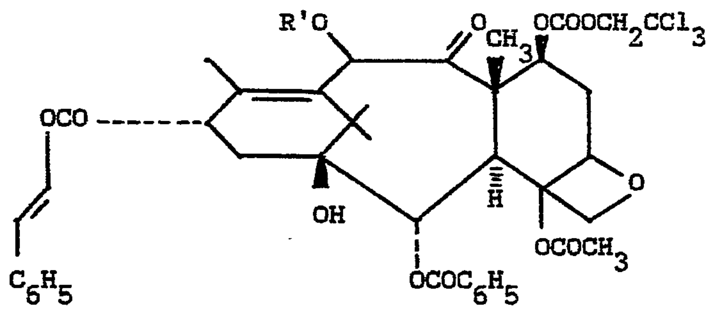

dans laquelle R', représente un radical acétyle ou trichloro-2,2,2 éthoxycarbonyle, dans un solvant organique tel que l'acétonitrile en présence de nitrate d'argent et d'une solution tertiobutanolique de tétraoxyde d'osmium à une température comprise entre 0 et 40°C, puis remplace par un atome d'hydrogène le ou les groupements trichloro-2,2,2 éthoxycarbonyle du produit de formule générale:

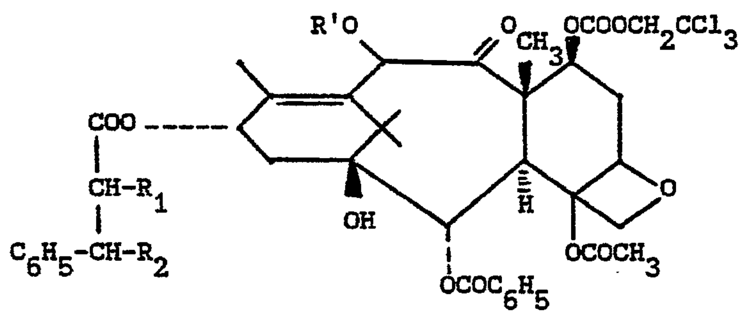

dans laquelle R' est défini comme ci-dessus et $R_1$ et $R_2$ sont définis comme dans la revendication 1, au moyen de zinc en présence d'acide acétique à une température comprise entre 30 et 60°C, et isole le produit obtenu.

2. Procédé de préparation d'un produit de formule générale:

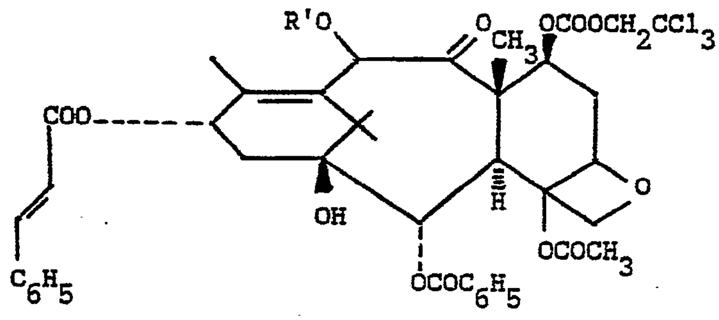

dans laquelle R' représente un radical acétyle ou trichloro-2,2,2 éthoxycarbonyle caractérisé en ce que l'on fait réagir l'acide cinnamique sur un produit de formule générale:

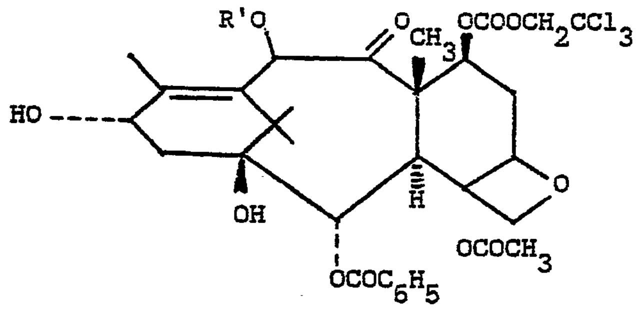

dans laquelle R' est défini comme précédemment en opérant dans un hydrocarbure aromatique tel que le benzène, le toluène ou les xylènes en présence d'un agent de condensation tel qu'un carbodiimide comme le dicyclohexylcarbodiimide ou un carbonate réactif comme le dipyridyl-2 carbonate et d'un agent d'activation tel que la diméthylaminopyridine à une température comprise entre 60 et 90°C.

**Patentansprüche für die Vertragsstaaten BE, CH, DE, FR, GB, IT, LI, LU, NL, SE**

1. Neues Derivat des Taxols der allgemeinen Formel:

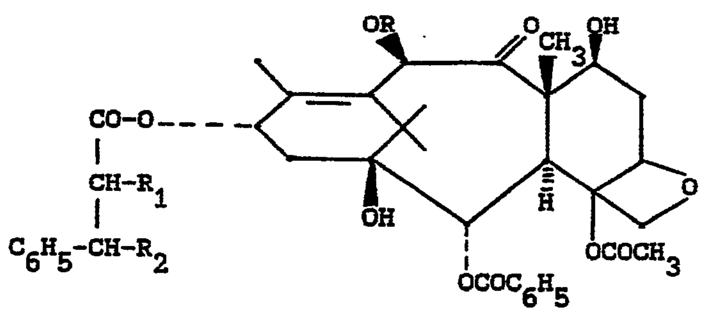

worin R ein Wasserstoffatom oder einen Acetylrest bedeutet, eines der Symbole $R_1$ oder $R_2$ einen Hydroxyrest darstellt und das andere einen tert.-Butoxycarbonylaminorest bedeutet, sowie seine stereoisomeren Formen und deren Gemische.

2. Verfahren zur Herstellung eines neuen Derivats des Taxols gemäß Anspruch 1, dadurch gekennzeichnet, daß man das Natriumsalz von tert.-Butyl-N-chlorcarbamat auf ein Produkt der allgemeinen Formel:

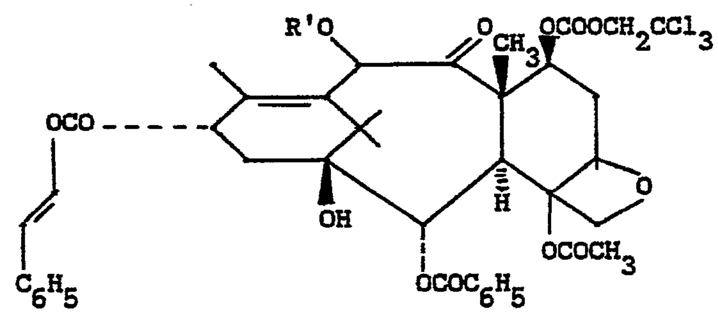

worin R′ einen Acetylrest oder 2,2,2-Trichlorethoxycarbonylrest bedeutet, in einem organischen Lösungsmittel wie Acetonitril in Anwesenheit von Silbernitrat und einer tert.-butanolischen Lösung von Osmiumtetraoxid bei einer Temperatur zwischen 0 und 40°C einwirken läßt, dann die 2,2,2-Trichlorethoxycarbonylgruppe(n) des Produkts der allgemeinen Formel:

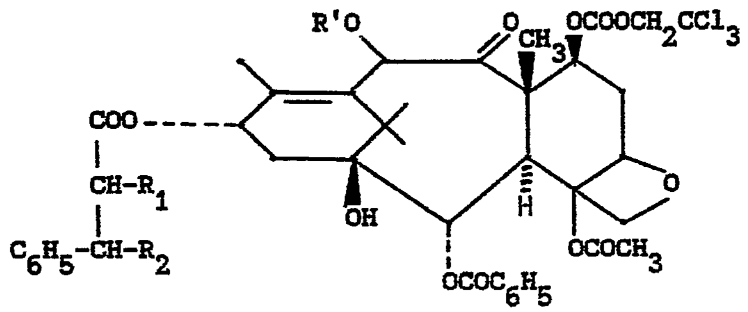

worin R′ wie vorstehend definiert ist und $R_1$ und $R_2$ wie in Anspruch 1 definiert sind, mittels Zink in Anwesenheit von Essigsäure bei einer Temperatur zwischen 30 und 60°C durch ein Wasserstoffatom ersetzt und das erhaltene Produkt isoliert.

3. Verfahren zur Herstellung eines Produkts der allgemeinen Formel:

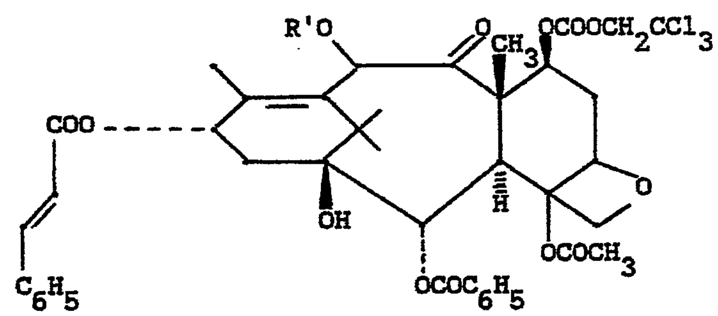

worin R′ einen Acetylrest oder 2,2,2-Trichlorethoxycarbonylrest bedeutet, dadurch gekennzeichnet, daß man Zimtsäure auf ein Produkt der allgemeinen Formel:

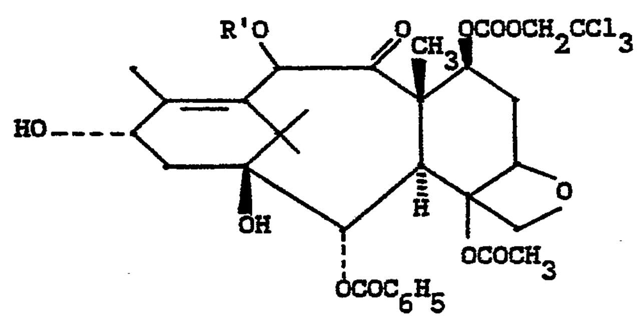

worin R′ wie vorstehend definiert ist, einwirken läßt, indem in einem aromatischen Kohlenwasserstoff wie Benzol, Toluol oder den Xylolen in Anwesenheit eines Kondensationsmittels wie einem Carbodiimid wie

Dicyclohexylcarbodiimid oder eines reaktionsfähigen Carbonats wie Dipyridyl-2-carbonat und einem Aktivierungsmittel wie Dimethylaminopyridin bei einer Temperatur zwischen 60 und 90°C gearbeitet wird.

4. Pharmazeutische Zusammensetzung, dadurch gekennzeichnet, daß sie eine ausreichende Menge eines Produkts gemäß Anspruch 1 zusammen mit einem oder mehreren pharmazeutisch annehmbaren, inerten oder pharmakologisch aktiven Verdünnungs- oder Hilfsmitteln enthält.

**Patentansprüche für die Vertragsstaaten AT, ES, GR**

1. Verfahren zur Herstellung eines neuen Taxolderivats der allgemeinen Formel:

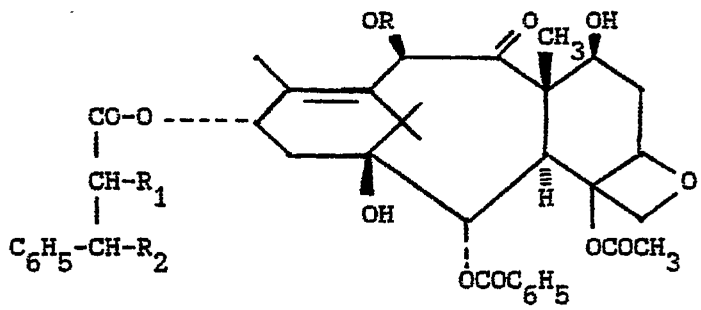

in welcher R ein Wasserstoffatom oder einen Acetylrest darstellt, eines der Symbole $R_1$ oder $R_2$ einen Hxdroxyrest darstellt und das andere einen tert.-Butoxycarbonylaminorest darstellt, sowie von dessen stereoisomeren Formen und ihren Mischungen, dadurch gekennzeichnet, daß man das Natriumsalz von tert.-Butyl-N-chlorcarbamat auf eine Verbindung der allgemeinen Formel:

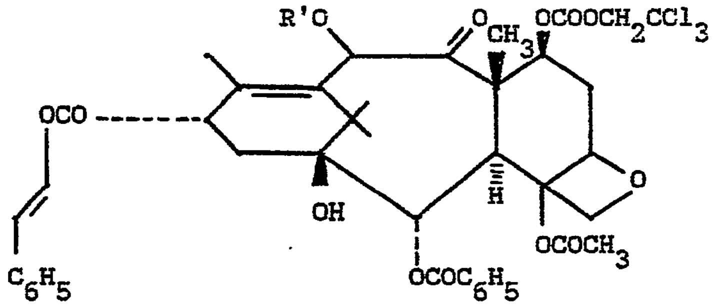

in welcher R' einen Acetyil- oder 2,2,2-Trichloräthoxycarbonylrest darstellt, in einem organischen Lösungsmittel, z.B. Acetonitril, in Gegenwart von Silbernitrat und einer Lösung von Osmiumtetraoxid in tert.-Butanol bei einer Temperatur zwischen 0 und 40°C einwirken läßt und dann das oder die 2,2,2-Trichloräthoxycarbonylgruppen des Produkts der allgemeinen Formel:

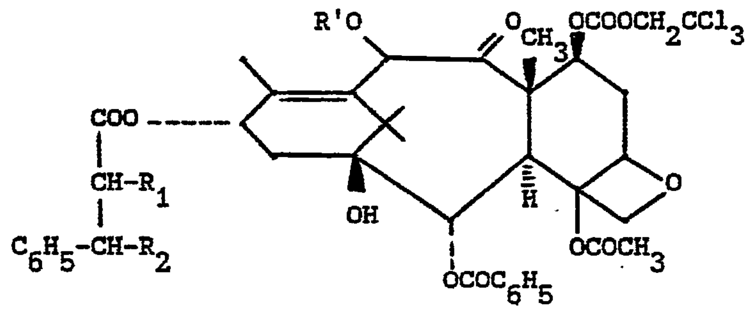

in welcher R' die obige Bedeutung hat und $R_1$ und $R_2$ die im Anspruch 1 angegebene Bedeutung haben, mit Zink in Gegenwart von Essigsäure bei einer Temperatur zwischen 30 und 60°C durch ein Wasserstoffatom ersetzt und das erhaltene Produkt isoliert.

2. Verfahren zur Herstellung einer Verbindung der allgemeinen Formel:

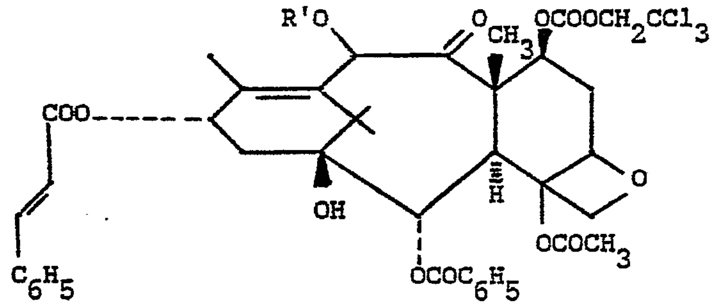

in welcher R′ einen Acetyl- oder 2,2,2-Trichloräthoxycarbonylrest darstellt, dadurch gekennzeichnet, daß man Zimtsäure mit einer Verbindung der allgemeinen Formel:

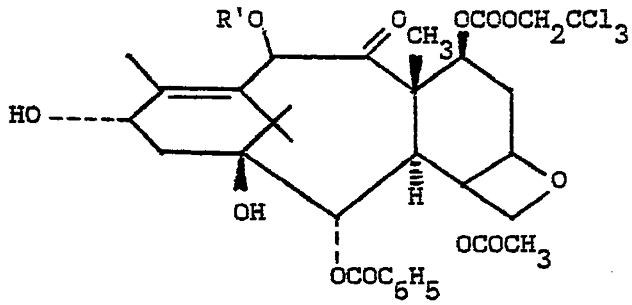

in welcher R′ die obige Bedeutung hat, umsetzt, indem man in einem aromatischen Kohlenwasserstoff, z.B. Benzol, Toluol, den Xylolen, in Gegenwart eines Kondensationsmittels, z.B. eines Carbodiimids wie Dicyclohexylcarbodiimid, oder eines reaktionsfähigen Carbonats wie 2-Dipyridylcarbonat und eines Aktivierungsmittels wie Dimethylaminopyridin bei einer Temperatur von 60 bis 90°C umsetzt.

**Claims for the contracting states: BE, CH, DE, FR, GB, IT, LI, LU, NL, SE**

1. A new taxol derivative of general formula:

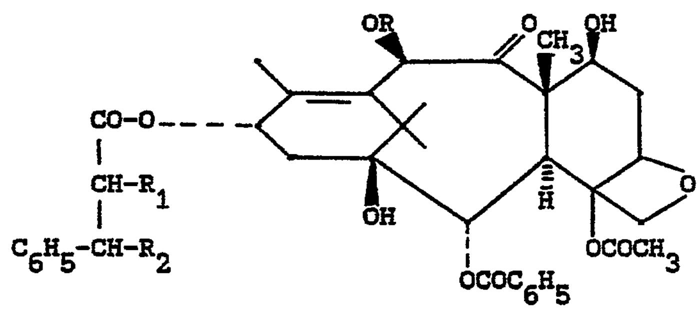

in which R represents a hydrogen atom or an acetyl radical, one of the symbols $R_1$ or $R_2$ represents a hydroxy radical and the other represents a tert-butoxycarbonylamino radical, and the stereoisomeric forms thereof and the mixtures thereof.

2. A process for the preparation of a new taxol derivative according to claim 1, wherein the sodium salt of tert-butyl N-chlorocarbamate is reacted with a product of general formula:

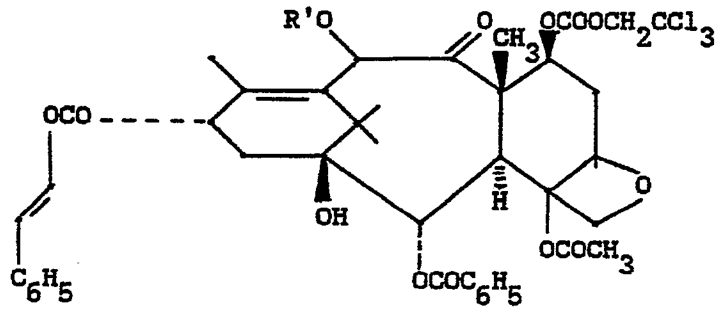

in which R′ represents an acetyl or 2,2,2-trichloroethoxycarbonyl radical, in an organic solvent such as acetonitrile in the presence of silver nitrate and a tert-butanolic solution of osmium tetroxide at a temperature between 0 and 40°C, and wherein the 2,2,2-trichloroethoxycarbonyl group(s) of the product of general formula:

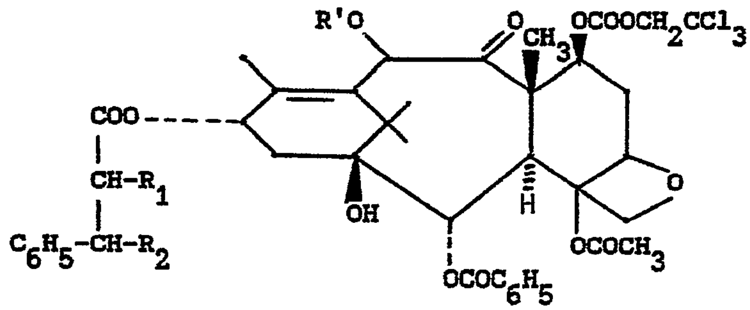

in which R′ is defined as above and $R_1$ and $R_2$ are defined as in claim 1, is (are) then replaced with a hydrogen atom using zinc in the presence of acetic acid at a temperature between 30 and 60°C, and the product obtained is isolated.

3. A process for the preparation of a product of general formula:

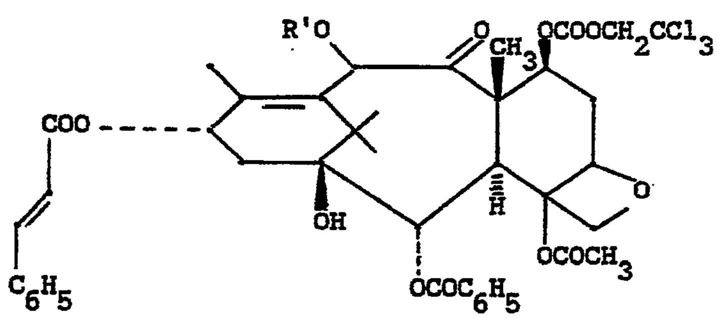

in which R′ represents an acetyl or 2,2,2-trichloroethoxycarbonyl radical, wherein cinnamic acid is reacted with a product of general formula:

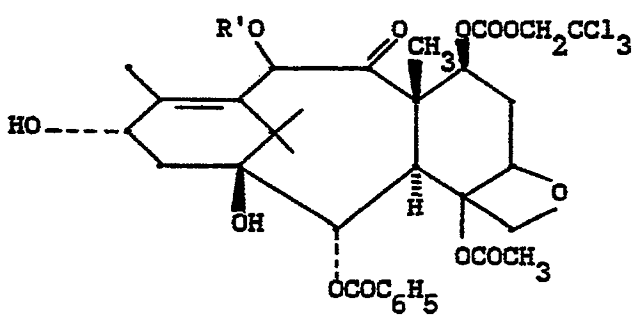

in which R′, is defined as above, operating in an aromatic hydrocarbon such as benzene, toluene or xylenes, in the presence of a condensing agent such as a carbodiimide, for example dicyclohexylcarbodiimide, or a reactive carbonate such as di-2-pyridyl carbonate and an activation agent such as a diaethylaminopyridine, at a temperature between 60 and 90°C.

4. A pharmaceutical composition which contains a sufficient quantity of a product according to claim 1 combined with one or more pharmaceutically acceptable, inert or pharmacologically active diluents or adjuvants.

**Claims for the contracting states: AT, ES, GR**

1. A process for the preparation of a new taxol derivative of general formula:

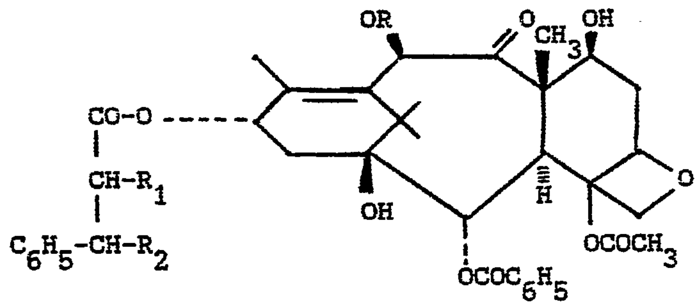

in which R represents a hydrogen atom or an acetyl radical, one of the symbols $R_1$ or $R_2$ represents a hydroxy radical and the other represents a tert-butoxycarbonylamino radical, and the stereoisomeric forms thereof and the mixtures thereof, wherein the sodium salt of tert-butyl N-chlorocarbamate is reacted with a product of general formula:

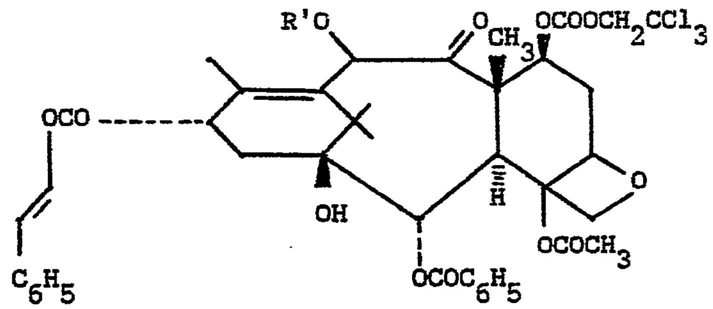

in which R′ represents an acetyl or 2,2,2-trichloroethoxycarbonyl radical, in an organic solvent such as acetonitrile in the presence of silver nitrate and a tert-butanolic solution of osmium tetroxide at a temperature between 0 and 40°C, and wherein the 2,2,2-trichloroethoxycarbonyl group(s) of the product of general formula:

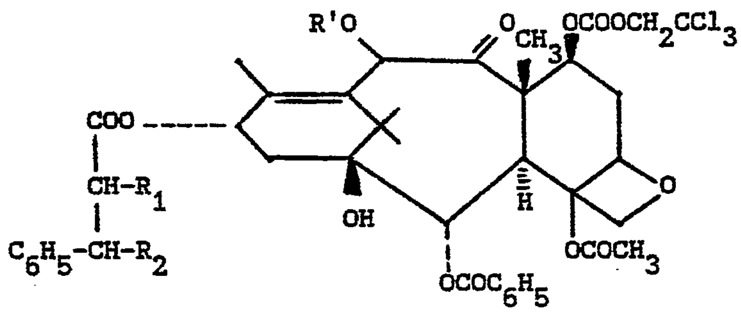

in which R′ is defined as above and $R_1$ and $R_2$ are defined as in claim 1, is (are) then replaced with a hydrogen atom using zinc in the presence of acetic acid at a temperature between 30 and 60°C, and the product obtained is isolated.

2. A process for the preparation of a product of general formula:

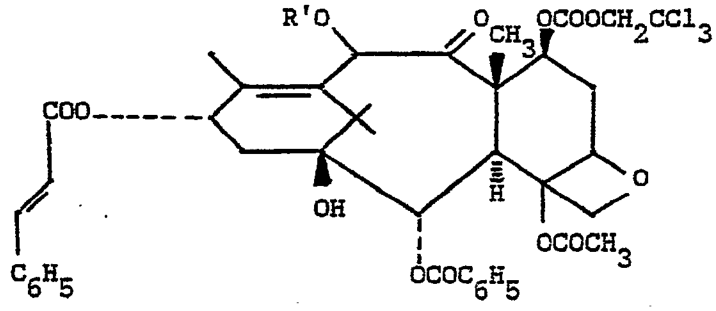

in which R′ represents an acetyl or 2,2,2-trichloroethoxycarbonyl radical, wherein cinnamic acid is reacted with a product of general formula:

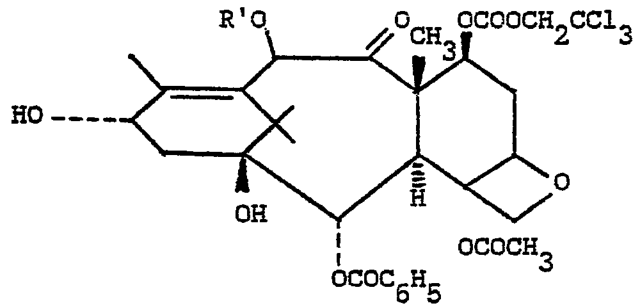

in which R′ is defined as above, operating in an aromatic hydrocarbon such as benzene, toluene or xylenes, in the presence of a condensing agent such as a carbodiimide, for example dicyclohexylcarbodiimide, or a reactive carbonate such as di-2-pyridyl carbonate and an activation agent such as a dimethylaminopyridine, at a temperature between 60 and 90°C.